(19) Europäisches Patentamt
European Patent Office
Office européen des brevets

(11) EP 2 730 924 A1

(12) EUROPEAN PATENT APPLICATION

(43) Date of publication:
**14.05.2014 Bulletin 2014/20**

(51) Int Cl.:
***G01N 33/68*** $^{(2006.01)}$ ***G06F 19/24*** $^{(2011.01)}$

(21) Application number: **13192071.2**

(22) Date of filing: **08.11.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **08.11.2012 US 201213672649**

(71) Applicant: **Veterinary Diagnostics Institute, Inc.**
**Simi Valley, CA 93063 (US)**

(72) Inventor: **Ringold, Randy**
**West Hills, CA 91304 (US)**

(74) Representative: **ZBM Patents - Zea, Barlocci & Markvardsen**
**Zea, Barlocci & Markvardsen**
**Plaza Catalunya, 1**
**08002 Barcelona (ES)**

# (54) Method and system for detecting underlying health affections using biomarkers in humans and animals

(57) The invention provides a method and system for developing and using diagnoses of one or more underlying health affections in humans or an animal specie using a plurality of biomarkers. A target one or more affections are defined and a set of biomarkers is selected. An index is computed based on the measured levels of the biomarkers. The biomarkers levels are discretized, and each discrete value is multiplied by a corresponding coefficient. The index scale is divided into ranges that are matched with health statuses. A subject's health affection status is subsequently determined by measuring the level of each biomarker in the set, computing the index and matching the index value to the predefined index scale.

EP 2 730 924 A1

**Description**

FIELD OF THE INVENTION

The invention relates to detecting disease in animals and humans; and particularly the invention provides a method and system for constructing a diagnosis for one or more particular health affections using a plurality of biomarkers, and further using the diagnosis to differentiate between the several underlying health affections regardless of the similarity in the apparent symptoms, and detect the propensity of an animal or human to develop a health affection.

BACKGROUND OF THE INVENTION

The level of biomarkers in body fluids is used in the process of detecting numerous health affections. Measuring the level of one or more specific biomarkers in the blood is typically a fast and relatively inexpensive means for diagnosing a disease or leading to the diagnosis thereof before prescribing other (more expensive and/or time consuming) tests such as radiological, cytological, histological and immunological tests etc.

However, many challenges face a medical (or veterinary) practitioner in selecting a panel of biomarkers to be tested for any specific case, and then in interpreting the results of the measured level of each biomarker in view of the symptoms the patient is exhibiting. The symptoms are generally only broad indicators of any particular disease, since some diseases, such as infection-related diseases, trigger symptoms in a patient at the onset of the disease or shortly thereafter, while other diseases, such as many types of cancer, trigger symptoms a considerable time after a tumor starts to develop. Diagnosing the underlying disease often requires testing for a panel of biomarkers, where some tests may be conducted to confirm a suspicion of a disease, while other tests may be conducted to rule out one or more other diseases.

Along with the challenge of choosing the panel of biomarkers for testing, there is the challenge of interpreting the test results. Some biomarkers are proven to be tightly correlated with the presence of a certain disease, while other biomarkers provide only a probability level a patient has a particular disease. For example, in humans sustained hyperglycemia is a good indicator of diabetes, while, for example, in dogs an increased level of thymidine kinase above a given threshold is an indicator of Hemangiosarcoma, which should be confirmed through histology.

Moreover, screening, diagnosing and/or monitoring a disease may involve any number of tests. According to current methods and systems, when using biomarkers the broader are the symptoms, the more tests are carried out. A practitioner uses his/her own experience to interpret the test results when using biomarkers to detect a disease, to follow the progression of a disease and/or to monitor the result of a treatment. The latter introduces a level of subjectivity in diagnosing test results, which may cause discrepancies between interpretations by the same person over time, among practitioners and even among entire health institutions.

Therefore, there is a need for a method and system for selecting a set of biomarkers and developing a method of use for detecting one or more target diseases and differentiating between the diseases to help a practitioner interpret the test results and potentially reveal the underlying affection or the propensity of a patient to develop a given disease.

**SUMMARY OF THE INVENTION**

The invention provides a method and system for constructing a medical (or veterinary) diagnosis using a plurality of biomarkers to reveal one or more particular health affections, and further to differentiate a particular underlying health affection regardless of the similarity in the apparent symptoms with other health affections. The diagnosis may also be used to reveal the propensity of an animal or human to develop a health affection at a later stage in life. The invention may be practiced in humans or animals such as domestic animal species.

Constructing a diagnosis method, according to the invention, starts by defining a target set of health affections in humans or an animal specie, selecting a set of biomarkers and measuring their levels in a group of subjects. The invention teaches how to compute a numerical value, i.e. an index, using the biomarker levels, then define ranges of the index on a scale, where each range may be matched with a subgroup of subjects segregated on the basis of their health status. Provided the latter method of segregation of subjects by health status, a practitioner may subsequently measure the level of biomarkers of a particular diagnosis in a patient, compute the index value for that particular patient and determine the status (or the type) of the underlying health affection of the patient by comparing the index value of the subject to the predefined ranges of the index.

A system according to the invention may be implemented as a computer program configured to receive input data (e.g., biomarker data and health status data etc.), and determine ranges for a particular diagnosis. The computer system may also receive the input for a particular patient, compute the index value and output the result of the diagnosis. The system may stand alone or be embedded in any diagnosis machine.

Currently, practitioners are faced with the difficulty of interpreting the results of biomarker data, particularly when comparing the progress of a disease, such as while monitoring a subject with a particular propensity of having a disease, or monitoring the health progress of patients following a treatment. The invention provides the latter practitioners tools for diagnosing an underlying health affection and monitoring the progress of a disease using numerical indicators for any particular situation.

## DESCRIPTION OF THE DRAWINGS

Figure 1 is a flowchart diagram representing steps involved in developing a method for detecting and/or differentiating one or more target diseases, in accordance with an embodiment of the invention.

Figure 2A is a flowchart of method steps involved in using a set of biomarkers in a diagnosis of one or more health statuses, in accordance with an implementation of the invention.

Figure 2B is a graphical representation of a continuous index scale and defined index ranges corresponding health statuses as taught by the invention.

Figure 3 shows a histogram of the age distribution of the cohort of dogs involved in one study.

Figure 4 shows a scatter plot representing cohort data for TK and CRP at six months status from the start of a study to develop a diagnosis method according to the invention.

Figure 5 shows plots of the Receiver Operating Characteristic (ROC) curves Sensitivity vs. one hundred (100) minus Specificity using TK alone and the two-biomarker implementation of the invention following confirmation of cancer in a subset of subjects.

Figure 6 is a block diagram representing components involved in the implementation of an embodiment of the invention.

## DETAILED DESCRIPTION OF THE INVENTION

The invention provides a method and system for detecting disease in animals and humans. The invention provides a method and system for developing and using a diagnosis targeting a particular one or more health conditions using a plurality of biomarkers, and further using the diagnosis to differentiate between several affections that may trigger similar health symptoms in a patient. Furthermore, the method and system according to the invention may be used to determine the propensity for an individual to develop a disease (e.g., one or more types of cancer), providing a practitioner the means for detecting a disease before the symptoms are visible and/or monitoring a disease post treatment.

In the following description, numerous specific details are set forth to provide a more thorough description of the invention. It will be apparent, however, to one skilled in the pertinent art, that the invention may be practiced without these specific details. In other instances, well known features have not been described in detail so as not to obscure the invention. The claims following this description are what define the metes and bounds of the invention.

### Terminology

Throughout the description, the terms individual, subject or patient may refer to an animal subject or a person whose biological data are used to develop and/or use an implementation of the invention. The subject may be normal (or disease-free) or showing any level of symptoms.

The term biomarker refers to any indicator in any body part (e.g., bodily fluid or tissue) that may be collected and the presence of which measured through any of its manifestations such as enzymatic activity, mass, concentration, cell count, cell shrinkage/shape, deoxyribonucleic acid (DNA) and/or ribonucleic acid (RNA) genetic level of expression or any aspect of the biochemical or the physiological markers that may be related to one or more health conditions. Moreover, for the purpose of designing health status indices (see below) a biomarker data may be any related data that may be considered for diagnosing a disease (or the probability of occurrence thereof) such as age, sex, any biometric data, genetic history (e.g., parent's health status or presence of any affection in the family) or any other data that may contribute to the diagnosis of a disease.

The term "index" is used throughout the disclosure to refer to a dependent variable that is calculated using two or more data inputs such as the level of a biomarker in the blood stream. A "neoplasia index" refers to an index that is computed with the goal of classifying subjects into groups based on cancer status. For example, a subject that may be apparently healthy (e.g., showing no signs of cancer), diagnosed with a malignant or a benign cancer or in any health status with regard to cancer, would have an neoplasia index value that reflects the health status, in accordance with embodiments of the invention.

The term "user" may be used to refer to a person, machine or a computer program acting as or on behalf of a person.

**General concept of the invention**

The main concept of the invention is that by selecting a set of biomarkers and measuring their levels in an animal or a human subject, it is possible to compute a numerical value, i.e. an index, using those levels, and to compare the index value to a predefined scale that characterizes the health status of the subject using index ranges. The scale may define two or more ranges of the index values, wherein each range indicates a level of one or more diseases. For example, in a subject showing general symptoms of inflammation, a predefined scale may define two or more ranges that may indicate the presence of cancer, an infection, both cancer and infection or other diseases.

The invention teaches developing any particular diagnosis by selecting a set of biomarkers, then measuring the level of each biomarker from each individual of a sample group of subjects. In addition, other diagnoses (e.g., cytological, histological and physiological tests, physical examination etc.) are carried out on the subjects to accurately establish the health status of each subject.

According to embodiments of the invention, the biomarkers data serve to compute the index values, while the health status data serve to define two or more health status categories (e.g., healthy, cancer, benign tumor, infection etc.). Ranges of index values are then defined providing efficient segregation of subjects into the two or more health status categories.

Subsequently, to provide a diagnosis to a patient, a set of biomarkers according to a particular diagnosis is collected and measured, then an index value is computed using the test data, and the index value is compared to the predefined index scale to match a health status category, which reveals the patient's health status.

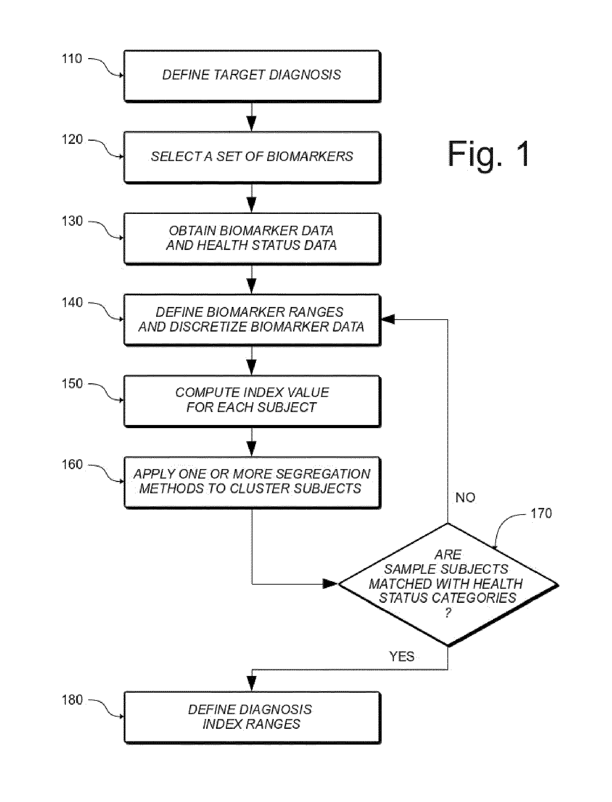

Figure 1 is a flowchart diagram representing steps involved in developing a method for detecting and/or differentiating one or more target diseases, in accordance with an embodiment of the invention. Step 110 represents defining a target diagnosis. A typical target diagnosis involves defining a disease (e.g., Infection, any type of Cancer etc.) or two or more diseases that may or may not display common symptoms. Any prior knowledge with regard to the target disease(s) may be considered, thus, the symptoms that accompany the disease, the severity of the symptoms, the speed at which the symptoms develop and any other aspect of the disease profile may be considered to define the target diagnosis.

Step 120 represents selecting a set of biomarkers for use in the diagnosis. Selecting a set of biomarkers may be based on previous knowledge of a correlation (be it positive or negative) between the level of a given biomarkers and the presence (or absence) of the one or more target diseases. For example, thymidine kinase may be used as a biomarker to detect any type of cancer since thymidine kinase is typically present in the cells undergoing cell division, which is the case of cancerous cells.

Step 130 represents collecting data from a group of subjects. The group of subjects may be a sample of subjects comprising normal subjects (i.e. healthy) showing none of the symptoms defined in Step 110, and affected subjects showing any level of severity of those symptoms. Bodily fluids, tissue or any other body sample may be appropriately collected in order to measure the level of each biomarker of the set of biomarkers defined at step 120.

In addition, the subjects undergo a plurality of tests, such as histological, radiological tests or any other test designed to establish the presence or absence of the target disease(s). Other tests may be conducted on each subject to either further confirm the disease or rule out other diseases that may share common symptoms.

Moreover, other non-disease related data may also be considered. The latter data comprise age, sex, any biometric data, genetic history (e.g., parent's health status or presence of any affection in the family) or any other data that may contribute to the diagnosis of a disease.

The outcome of step 130 is a set of data points that characterizes each subject individual data and its level of each biomarker in the set of data, and a health status that establishes whether each subject is a non-carrier or a carrier of one or more diseases and eventually the stage (or severity) of each disease. For example, when considering cancer, a subject may be classified as non-carrier of cancer, having a benign tumor, an early cancer stage or advanced cancer stage, and any given type of cancer. In the latter example, the set of biomarkers may comprise thymidine kinase, C-reactive protein and/or any other biomarker selected at step 110 to include in the development of the diagnosis method. The level of each biomarker may be expressed in any unit that characterizes the presence of the biomarker in the body. Thus, an enzyme may be characterized by the level of its enzymatic activity, a protein, a hormone or any other biomarker may be expressed by a concentration level such as its mass or moles per volume of tissue or bodily fluid.

Step 140 represents the process of finding range values for each biomarker. For example, when considering thymidine kinase as a biomarker for cancer, a first range of zero units per liter (0 U/l) through five units per liter (5 U/l), a second range of five units per liter (5 U/l) through eight units per liter (8 U/l), and a third range of eight units per liter (8 U/l) and above may be defined as ranges for that specific biomarker.

Step 140 also involves discretizing the data, which comprises attributing a score number to each previously defined range of a biomarker level. Using the example of the three (3) thymidine kinase ranges above, the first range may be attributed the value zero (0), the second range may be attributed the value one (1) and the third range may be attributed the value two (2).

The discretization may be carried on other non-disease related data such as age. In the latter example, age may be selected for the diagnosis as a factor in the increase of the probability of having a target affection. Thus, age may be discretized such that a person of 0 to 20 years of age is attributed a value of "0", a person of 20 to 40 years of age may be attributed a value "1" and a person over 40 years of age may be attributed a value of "2". Sex may be discretized as "1" and "0" for female and male, respectively.

Step 150 represents computing an index value for each subject as follows:

$$I = \sum_{i=1}^{i=N} C_i \cdot L_i \qquad (1)$$

where the index value "*I*" for each subject may be the sum of the product of the score level *"L"* (e.g., computed at step 140) and a coefficient "C" associated with the *"i^th"* data input for a number "*N*" of data inputs (e.g., biomarker level, age, biometric data etc.). The coefficient "C" may be determined empirically as shown below at steps 160 and 170.

Step 160 represents applying one or more methods for segregating subjects using the health status data and the computed index values. For example, the method of segregation may be the Receiver Operating Characteristic (ROC) curve analysis. ROC curve analysis is a well known method in the medical field for determining whether a correlation between the level of a biomarker may serve as an indicator of the presence of a health condition. The latter is possible for example when there is a strong correlation between the amount of a substance in the body (e.g., high cholesterol) and a health condition (e.g., sclerosis of blood vessels).

Using the ROC curve analysis on the index values of all subjects in the group, it is possible to determine whether there is a cutoff value capable of classifying individuals into groups matching their health status. For example, if subjects carrying a disease are labeled as positive and the non-carriers are labeled as negative, the ROC curve analysis may yield a threshold that classifies the subjects into an above and a below-threshold groups matching the health statuses carrier and non-carrier of the disease, respectively. There may be false positives and false negatives for each chosen cutoff value in the range of possible values. The rate of success in determining true positive cases is called "Sensitivity", whereas the rate of success in determining true negative cases is called "Specificity". Sensitivity and specificity for a plurality of cutoff values are computed. Sensitivity and Specificity are rates, and thus may be expressed in the range of zero (0) to one (1), or as a percentage from zero (0) to one hundred percent (100%). The results are plotted as Sensitivity values versus one (1) (or 100% depending on the unit of choice) minus the corresponding specificity. The area under the curve (AUC) reveals whether ROC analysis may be a valid classifier of the data: the closer the AUC is to 100%, the better classifier is the ROC analysis. On the contrary, the ROC analysis may not be considered for classification purposes if the AUC is closer to 50%, which is considered close to a random process. In general, the ROC method of analysis may be considered valid, if the AUC is at least 0.8.

Moreover, each threshold value yields a "Sensitivity" and "Specificity". In populations where where ROC analysis appears adequate, the "Sensitivity" curve decreases as the "Specificity" increases. At a particular threshold, the apex, the total of Sensitivity and Specificity is at a maximum. The apex is typically chosen as the threshold of classification if it yields a Sensitivity and Specificity each above 0.85, otherwise a threshold for Specificity and a threshold for Sensitivity may be respectively selected to yield a success rate of at least 0.85.

ROC analysis is one of any existing methods that may be utilized in embodiments of the invention to detect clusters in the data that define the clustering boundaries capable of segregating subjects into groups matching health status categories. For example, k-means clustering, hierarchical clustering, neural networks or any other clustering clustering method may be utilized in one or more embodiments of the invention. Furthermore, an embodiment of the invention may conduct the steps of Figure 1 using a plurality of methods of clustering the data to achieve the results of the invention. The final clustering method that may be retained in any particular embodiment of the invention may be the one that yields the highest success rate of the diagnosis.

Step 170 represents computing success scores of the method of segregating of subjects in the test group. If the success level of the segregation into health categories is not satisfactory (e.g., no statistical difference compared to a population drawn from a random process), the parameters for computing the index values are revised and the analysis is repeated at step 140. The process of searching for optimal parameters may be repeated until the result of classification of subjects reaches (or exceeds) an acceptable success rate. Otherwise, if no optimal parameters may be found, the result may indicate that the chosen set of biomarkers is unsuitable for segregating the subjects, based on the index method in question, into the proposed health status categories.

The search for optimal parameters may involve changing one or more boundary values for discretizing biomarker values, and/or the weight coefficients associated with each biomarker in computing the index value for each subject. The search method may be manual i.e. an expert practitioner may set the initial parameters and adjust them, through multiple iterations of computation, while considering the outcome of the success rate of classification of subjects into health status categories. Implementations of the invention may also use numerical methods for automatic search to optimize parameters. Such methods comprise brute force search, where a large number of values of parameters and combinations thereof are tested. The numerical methods for determining optimal values may use gradient descent search, random walk search or any other mathematical method for searching for optimal parameters in order to achieve the goal of maximizing the success rate of the classification of subjects into correct corresponding health status categories.

Computer programs for conducting a search, in accordance with an implementation of the invention, require ordinary skills in the art of computer programming. Moreover, existing computer programs may be adapted (through a programming scripting language) to carry out a search process in an implementation of the invention. Computer programs include such programs as Mathematica™, Matlab™, Medcalc™, or any other available computer program may be used.

Step 180 represent the final step of determining the final parameters (or range thereof) that may be used in a diagnosis of the target disease(s). The optimal parameters include the coefficient associated with each biomarker, the number of ranges and the boundary values that define the ranges for each biomarker. Step 180 also includes determining the index range boundaries that define the categories as defined by the health status of subjects. The latter parameters may be used in systems for diagnosing whether a subject is a carrier of the a disease, as will detailed below in the method of use.

The invention provides a means for facilitating the display and read out of the results by defining the boundaries between ranges as discrete values for ease of use. For example, a scale comprising two health statuses, such as "disease present" and "disease not present", may be defined has having a discrete boundary, such as one "1", where the scale range lower than "1" may be mapped to "disease not present" status, while the scale range greater than "1" is mapped to "disease present" status.

Defining range boundaries as discrete values may be carried out during the search for the optimal parameters (as described above). The discrete range boundary values may also be provided computationally (e.g., using multipliers and offsets) subsequent to determining the optimal parameters.

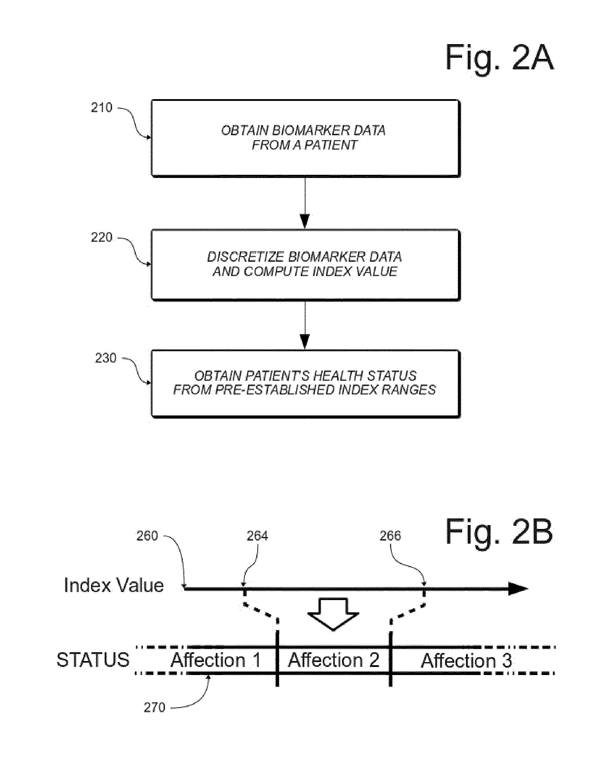

Figure 2A is a flowchart representing method steps involved in using a set of biomarkers in a diagnosis of one or more health statuses, in accordance with an implementation of the invention. Provided a set of pre-established optimal parameters that yield an acceptable success rate for classifying subjects into health categories based on a computed index from biomarkers, the invention provides a method and system for testing whether a new patient is likely a carrier of a suspected disease using biomarkers. Step 210 represents obtaining data from a patient. Similarly to step 130 and depending on the specific set of biomarkers involved in a diagnosis, bodily fluids, tissue and any other data necessary for the diagnosis are collected and the level of each biomarker is assessed.

Step 220 represents computing an index value for the patient. Provided the discretization boundary values for each biomarker, the level of each biomarker is converted into a score value, and provided the coefficient associated with each biomarker, the index value for the patient may be computed using equation (1).

Step 230 represents determining a patient’s health status group. The patient’s computed index value is compared to that of the established boundary values for health status categories. As described above, the established mapping between index values allows for ascertaining the health condition of a patient using its own index value.

Figure 2B is a graphical representation of a continuous index scale and defined index ranges corresponding to health statuses as taught by the invention. Line 260 represents a continuous scale of index values. Health status scale 270 represents the health status categories for which the diagnosis method was initially developed in accordance with the teachings of the invention. The health status scale may define two (2) or more health statuses, such as, in the case of cancer, non-carrier, benign tumor carrier and cancerous tumor carrier. Index values 264 and 266 may define the boundaries to read out the health status of a patient in question. Thus, a patient’s index value that is less than about boundary 264 would indicate the patient in question is in a first health status category, an index value greater than about boundary 264 and less than about boundary 266 would indicate the patient is in a second health category while an index value greater than boundary 266 would indicate that the patient is in a third health status category. For example, a patient’s index value may be within the range that matches the group of non-carriers of cancer, or the group of carriers of a benign tumor or the group of carriers of cancer.

The method steps as described in Figures 1, 2A and 2B may be carried out manually, i.e. a user may collect the data, compute the index value, then compare the the index value to a pre-defined set of ranges to obtain the health status category of a patient and/or the method steps may be implemented in a machine (e.g., digital computer) that carries out any or all of the steps of obtaining the data, computing the index value, obtaining the health status category and displaying/communicating the health status category to a user.

An embodiment of the invention may be implemented in a way where the biomarkers data considered for developing a target diagnosis are collected in (healthy) subjects showing no symptoms of the target affection. By monitoring the subjects over time and determining which subjects develop an ailment, the invention allows for building a diagnosis (or a predictor index) for revealing the propensity of a subject to develop a target affection in a future time based on current biomarker data.

The benefits of developing an index-based scoring system, in accordance with one or more implementations of the invention, are numerous. The teachings of the invention allow a practitioner to compare results obtained from different individuals using a plurality of data combined in an index. For assessing progress in an individual (e.g., monitoring health condition during or post-treatment), a practitioner may conduct the tests using several biomarkers and follow the variations of the index values. For assessing the risk factors for an individual to develop a given disease, a practitioner may determine a range of index values and/or a variation thereof over time that may be indicative of the development of the disease. For example, some dog breeds are more susceptible than others to developing certain types of cancer. The index values provided by a diagnosis, in accordance with an implementation of the invention, may be utilized to spot those individuals that may be in the process of developing a cancer at an early stage.

Moreover, since a plurality of implementations of the invention may be developed for the diagnosis/detection of various aliments, a patient may be subjected only once to a test of a superset of biomarkers that would include biomarkers from several target diagnoses. By measuring the level of various biomarkers, more than one index may be computed at any time. The result is that each patient may be represented in a multidimensional space of indices that characterizes the state of the patient. Thus, a practitioner is provided a means to assess the probability for the patient to have one ailment versus another ailment when both present common symptoms.

Cancer Detection in Dogs using TK and CRP

An embodiment of the invention targets the diagnosis of cancer in a typical patient i.e. showing general symptoms of inflammation while failing to reveal the underlying affection.

There has been a long standing and studied relationship between cancer and inflammation. The inflammatory response orchestrates host defenses to infection, trauma, toxins, or other tissue damaging events and mediates tissue repair and regeneration. Epidemiological evidence points to a connection between inflammation and a predisposition for the development of cancer, i.e. long-term inflammation leads to the development of dysplasia. Thus, while acute inflammation is normally tightly controlled and part of the healing process, chronic inflammation may be associated with a number of diseases including cancer.

In cancer, there is evidence that inflammation plays an essential role at each stage of the disease (initiation and proliferation), and both tumor and inflammatory cells are able to directly or indirectly either inhibit or stimulate tumor growth. The effectiveness of tumor development has been demonstrated to correlate directly with the degree of the inflammatory reactions, and it seems that there are interactions between the cytokines produced in response to inflammatory reactions and tumor growth and even indications that inflammatory cytokines favor tumor promotion. Furthermore, with the assistance of inflammation, tumor cells infiltrate neighboring tissues, enter into the bloodstream, migrate, and establish remote colonies i.e. metastases.

With the inflammatory process initiating the acute-phase reaction the generation of acute-phase proteins (APP) occurs. C-reactive protein (CRP) is a major APP and has been shown to be an effective measure of general inflammation. The concentration of CRP or any serum APP level correlates to both the severity and duration of the inflammatory stimuli.

Thymidine kinase type 1 (TK) is a cytosolic enzyme involved in DNA synthesis through the so called "salvage pathway" for thymidine biosynthesis, in which deoxythymidine is converted to deoxythymidine monophosphate, leading to its eventual incorporation into DNA. Cellular TK activity is closely correlated with the DNA synthesis phase of the cell cycle. As such, its expression is restricted to proliferating cells, and thus is often more highly expressed in malignant cells, which are characterized by dysregulated proliferation.

A method according to the invention may use the measurement of the biomarkers thymidine kinase (TK) and c-reactive protein (CRP) to diagnose whether a patient is affected by cancer. In accordance with the teachings of the invention, it should be implicit that CRP is used as a biomarker that represents the family of biomarkers involved in acute-phase reaction.

TK and CRP levels may be assessed through any available method for measuring their levels, whether directly or indirectly.

In accordance with the method of the invention described in Figure 1, an embodiment of the invention targets the detection of cancer using two or more biomarkers. To the latter end, a study involving 356 dogs was conducted to evaluate a dual biomarker method. At the start, the recruited dogs were "apparently healthy" i.e. with no overt signs of illness or history of cancer. The subjects are monitored for their health status. Over a period of time, the health status (e.g., presence versus non-presence of cancer) is matched with the levels of the biomarkers as measured initially in each subject. The goal is to establish a method, in accordance with an embodiment of the invention, for classifying subjects into two groups: one group comprising subjects that have developed cancer versus the rest of the subjects that remained unaffected by cancer. Thus, subsequently by measuring the level of the biomarkers in a subject, the method allows a practitioner to use the results as a diagnosis of current health status and/or as a predictor of the risk that a subject would eventually develop cancer.

Out of the 356 dogs recruited for the study, 378 dogs were enrolled and 22 disqualified due to prior history of cancer or inadequate specimen to complete analysis. The details of the cohorts breed and recruitment is shown in Table 1, and the cohort’s sex distribution is shown in Table 2. In the latter study the biomarkers chosen were thymidine kinase (TK) and a canine-specific c-reactive protein (c-CRP). After the initial measurement of the biomarkers, the subjects were monitored and tested for cancer over a period of several months.

Table 1: Breed details of the cohort

| Breed | Total | Total Disqualified | Total Included |
|---|---|---|---|
| **German Shepherd** | 173 | 11 | 162 |
| **White Shepherd** | 8 | 0 | 8 |
| **Golden Retriever** | 193 | 11 | 182 |
| **Portuguese Water dog** | 4 | 0 | 4 |
| **Total Dogs** | 378 | 22 | 356 |

Table 2: Sex distribution of the cohort

| Sex | Total | Total Disqualified | Total Included |
|---|---|---|---|
| **Female** | 84 | 4 | 80 |
| **Female Spaded** | 111 | 6 | 105 |
| **Male** | 97 | 6 | 91 |
| **Male Neutered** | 74 | 5 | 69 |
| **N/A** | 12 | 1 | 11 |
| **Total** | 378 | 22 | 356 |

Table 3 shows the types of cancer as confirmed through histological or cytological tests, or by observation when the subjects

**Table 3: Cancer types detected in the cohort**

| | Cancer Types | Total |
|---|---|---|
| **Histological/ Cytological Conformed** | • Leukemia<br>• Hemangiosarcoma | **6** |
| **Cancers** | • Sarcoma<br>• Lymphoma<br>• Hemangiosarcoma<br>• Single Cell Carcinoma | |
| **Observational Based** | • Hemangiosarcoma<br>• Lymphoma<br>• Hemangiosarcoma<br>• Parathyroid | 4 |
| | **Total Cancers** | 10 |

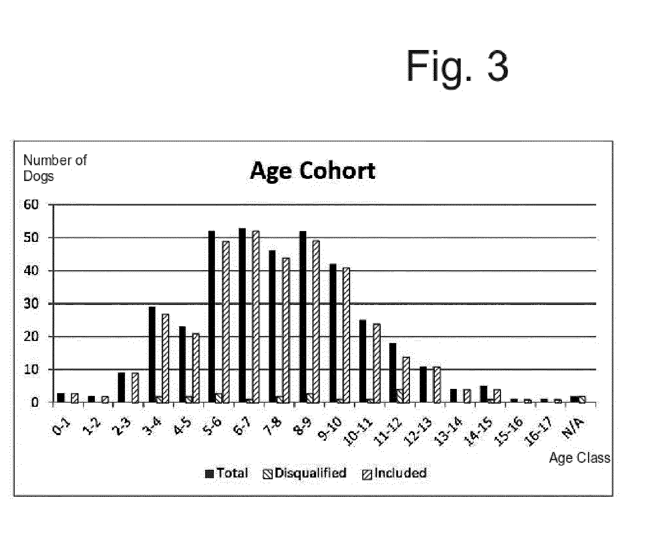

Figure 3 shows a histogram of the age distribution of the cohort of dogs involved in the study. Figure 3 shows the distribution of the total number of subjects in each class (i.e., age group) involved in the study, the number of disqualified subjects and the number that were included in the development of the cancer detection method.

The measurement of Thymidine kinase enzymatic activity in samples of blood plasma is described in US Patent 8,097,432 B2, which is included herewith in its entirety by reference. In brief, a blood plasma is separated from a sample of blood to be tested for TK activity. The plasma is introduced into a solution containing an analog of deoxythymidine nucleotide and a phosphate donor. The product of the enzymatic activity is then measured using an immunoassay.

The measurement of CRP may be carried out using any available method for extracting and measuring protein concentration in a bodily fluid or tissue. The latter methods comprise using centrifugal force, electrophoresis, chromatography, immuno-binding assays and any available method for measuring the concentration of a protein.

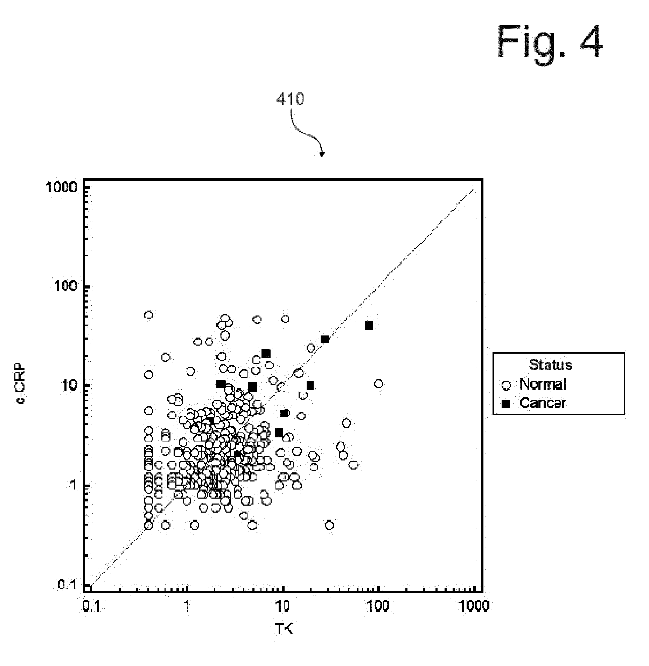

Figure 4 shows a scatter plot representing cohort data for TK and CRP at six month status from the start of the study. Plot 410 shows data points defined by the level of TK (abscissa), level of CRP (ordinate) and whether the subject was had cancer (square) or did not (circle). The scatter plot visually reveals that the dogs affected by cancer (as represented by squares) have a relatively high levels of both c-CRP and TK, and that these two biomarkers are correlated (follow the diagonal line) pointing to the association of inflammation with cancer.

Using TK and CRP in the latter study, and in accordance with the invention as described by formula (1) and Figure 1, a two-parameter neoplasia index may be computed, by applying equation (1), as follows:

$$N_2 = a_2 \cdot dTK + b_2 \cdot dCRP \qquad (2)$$

Where "$N_2$" denotes the Neoplasia index in a two (2) parameter model using TK and CRP, and where "$a_2$" and "$b_2$" denote the coefficients associated with TK and CRP, respectively. "dTK" and "dCRP" denote the discrete score value matched with a range of TK level and CRP level, respectively. As described above, TK level may be represented by the level of its enzymatic activity, whereas CRP may be represented by its mass (or moles) per volume of blood plasma.

As described above, the level of the biomarkers was measured and discretized. A discrete score assigned to ranges of the levels for each biomarker. Table 4 shows the detail of the discretization scores corresponding to ranges for each of TK and CRP level.

Table 4: Discrete scores for TK and CRP ranges

| TK (U/L) | c-CRP (mg/L) | Score |
|---|---|---|
| 0 to 1.7 | 0 to 3.9 | 0 |
| 1.8 to 4.0 | 4.0 to 9.5 | 1 |
| 4.1 to 7.0 | over 9.5 | 2 |
| over 7.1 | | 3 |

In the latter two-biomarker method, the coefficients "$a_2$" and "$b_2$" of formula (2) are given the values 2.1 and 1.6, respectively, when using the method for screening. When the method is used in a group of patients showing signs cancer (see below), the coefficients "$a_2$" and "$b_2$" of formula (2) are given the values 1.76 and 2.32, respectively.

Using the latter coefficients in formula (2), an index value lower than "1" would indicate low probability a patient has cancer versus a value greater than "1", which would indicate that the patient has cancer.

In the latter study, the subjects were followed over time, and tests were carried out at four (4) months, and six (6) months from the initial test. Other (histological) tests were also carried out to confirm the presence of cancer.

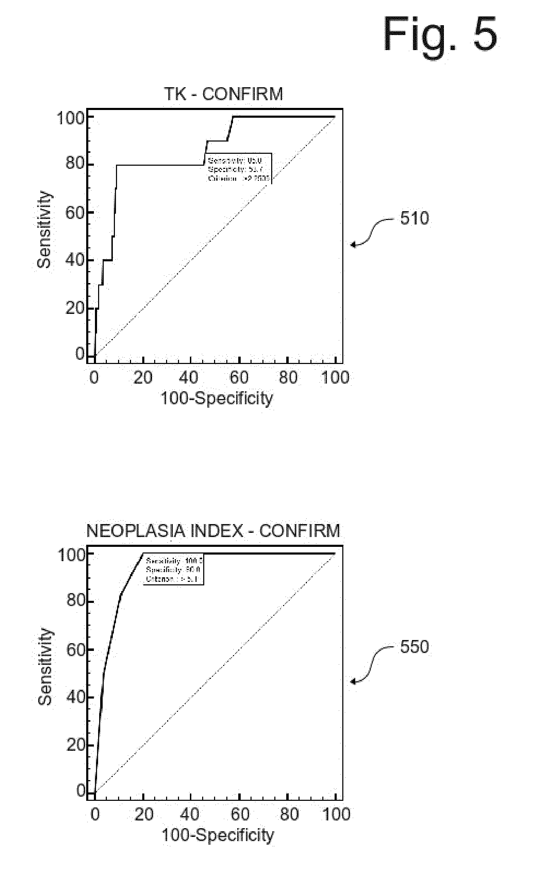

For comparison of the performance of the method provided by the invention versus previous methods that relied solely on a single biomarker (e.g., TK), Receiver Operating Characteristic (ROC) analysis has been carried out using TK alone or the two-biomarker implementation of the invention. The ROC curves representing Sensitivity vs. one hundred (100) minus Specificity is plotted in Figures 5, 6 and 7, and the area under the curve is computed and shown in Table 5.

Table 5: ROC AUC for TK vs. Neoplasia Index

| Status | TK Only | Neoplasia Index (TK&CRP) |
|---|---|---|
| **Confirmed Cancer** | 0.783 | 0.941 |
| **4-Months Status** | 0.889 | 0.970 |
| **6-Months Status** | 0.826 | 0.930 |

Figure 5 shows plots of the Receiver Operating Characteristic (ROC) curves Sensitivity vs. one hundred (100) minus Specificity using TK alone and the two-biomarker implementation of the invention following confirmation of cancer in a subset of subjects. Plot 510 shows the plot of the ROC curve for TK. The area under the curve (AUC) is 0.783 (or 78.3%). At value level of TK of 2.25 U/l or greater, the Sensitivity is .85 (or 85%), and the Specificity is .537 (or 53.7%). Plot 550 shows the ROC curve for the index computed using formula 2 with discrete values using table 4. The UAC in plot 550 is 0.941 (or 94.1%). Using the threshold value for the index of -5.1, the Sensitivity is 100% and the Specificity is 80%. Therefore, the predictive power of the method is greater when using the two-biomarker neoplasia index than by using TK alone to diagnose cancer dogs.

**System for developing and using a multi-biomarker index**

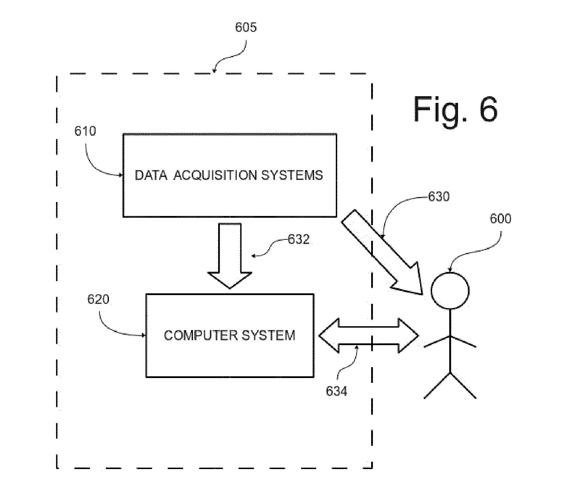

Figure 6 is a block diagram representing system components for implementing the development of use of diagnoses in accordance with an embodiment of the invention. The invention teaches the method steps described in Figures 1 and 2 as a general implementation of the development and use of diagnostic indices for any one or more target health affections in humans and/or animals. Furthermore, the invention teaches two-biomarker and three-biomarker methods for segregating canine subjects into groups affected by cancer, sepsis, SIRS. A system implementing an embodiment of the invention comprises one or more components for collecting data, one or more components for analyzing data, one or more components for communicating data with users.

Block 610 represents the data acquisition layer of any system that implements an embodiment of the invention. The system may be any system required for the acquisition of the biological data that may be associated with any particular target health affection for diagnosis or the development of a diagnosis thereof. For example, the biological data may require the measurement of the level of a particular substance in the blood (or in any other bodily fluid) and/or in an organ tissue. The substance may be a protein, a peptide, any type of hormone or any other molecule or ion the measurement of which may be relevant to the diagnosis of a particular health affection or the development of diagnosis thereof. In the case where a biomarker is a biological substance, Block 610 represents the necessary laboratory equipment for collecting biological samples and processing the samples in order to obtain the biological data required for a particular embodiment of the invention. For example, measuring a protein level in the blood requires many steps comprising collecting blood from subjects, separating the portion of blood that contains the protein (e.g., using a centrifuge), purifying the protein, submitting the a purified solution of the protein to a calibrated assay (e.g., using marked antibodies), or any other step that may yield the concentration of the protein in the blood sample. In other instances, the protein may be an enzyme, in which case it may be desirable to measure the concentration of the protein through its enzymatic activity level.

One with ordinary skills in the art of medical or veterinary diagnosis is able to recognize the laboratory method steps, and laboratory equipment represented by block 610 for collecting biological sample, extracting the pertinent biomarker, and measuring its level.

As described above in the definition of term "biomarker", the biological data may be any type of data that be involved in diagnosing a specific health affection whether the biological data may be assessed using a biological sample from the subject's body or through observational assessment of symptoms. For example, biological data comprise body temperature (e.g., in fever cases) heart beat rate, the number of siblings or parents having an affection (e.g., in cases of inherited affections), the elapsed time since the first signs of a disease started to show symptoms or any other non-substance related measurement that may be obtained from a patient and that may be considered in a diagnosis. Block 610 represents the tools and equipments necessary for collecting the biological data, which is accessible to one with ordinary skills in the pertinent art.

Block 620 represents a computer system for implementing and executing computer program instructions following the teachings of the methods developed in an embodiment of the invention. The computer system is any analog and/or digital computer capable of being configured to take input data, execute some or all of the method steps of the invention and provide the result of such execution to a user.

The computer system 620 may be a digital computer having a digital processor, a memory, a data transfer bus, a storage medium and any electronic communication means that allows the computer to receive and send data through display and communication to and from users and/or other machines. In embodiments of the invention, the computer may be embedded (as symbolized by block 605) in a device for carrying out medical (or veterinary) tests. Thus, a mobile and/or portable system comprises a device configured to collect the data such as determining the level or one or more biomarker level in a blood sample, and a computer system for carrying the steps of the invention. The device may be enabled with display means such that the results are communicated to a user.

In other embodiments of the invention, the computer 620 may stand alone, such as a computer system that is detached from any particular a device, while being capable of receiving data through direct communication (e.g., user interface), and/or remote communication means (e.g., networked data transfer).

The input data may be any of the biological and non-biological data, described above, that may be entered to the computer automatically through one or more links 632, or through a user interface 634 provided to a user 600. For example, a practitioner 600 may obtain the biological data from the data collection system through one or more communication (or interface links) 630, then enter the data into the computer system through the computer interface 634.

The practitioner may also enter in the computer system further configuration data, such as range boundaries for data discretization, optimization method or any other configuration data to conduct a search for optimal parameters. The computer system comprises program instructions to conduct a search for optimal parameters. Computer system 620 contains program instructions the execution of which allows to discretize new data (e.g., from a patient) compute one or more indices, then compare the new data to previously generated (or stored data) in order to provide a diagnosis.

In embodiments of the invention, a computer program as well as the data for any particular diagnosis method, in computer system 620, may be replicated from one machine to another, thus, allowing the diagnosis programs to be replicated to any number of other machines. For example, in portable blood test devices, a computer program may be configured to process the data and provide fast diagnosis using pre-stored diagnosis parameters.

However, the data acquisition system may be separated and remotely located and may serve the practitioner remotely. For example, the computer program may be implemented on a central unit that may collected biological data from a plurality of data acquisition systems (e.g., using computer networks), and serve client machines with diagnoses as they may be entered remotely. Furthermore, the collection of data from a plurality of client data acquisition system may serve to further refine the diagnosis program as more and more data become available.

Thus, a method and system for selecting a set of biomarkers and developing a method of use for detecting one or more target diseases and differentiating between the diseases to help a practitioner interpret the test results and potentially reveal the underlying affection or the propensity of a patient to develop a given disease.

For reasons of completeness, various aspects of the invention are set out in the following numbered clauses:

Clause 1. A method for developing a diagnosis method of affections in an animal species using a plurality of biomarkers, comprising the steps of:

defining a set of health affections;

defining a set of biomarkers;

selecting a group of subjects;

assessing a presence of a health affection of said set of health affections in each individual in said group of subjects;

assessing a level of each biomarker in said set of biomarkers;

computing an index value using said level of said each biomarker; and

obtaining a set of ranges of said index value, wherein at least one range of said set of ranges comprises at least eighty percent (80%) of a subgroup of individuals, from said group of subjects, that are affected by said health affection from said set of health affections.

Clause 2. The method of clause 1, wherein said step of assessing said presence of said health affection further comprising performing at least one biological test of each of said individual to determine said presence.

Clause 3. The method of clause 1, wherein said step of assessing said level of said each biomarker further comprising measuring the concentration of at least one protein in the blood stream of said individual.

Clause 4. The method of clause 1, wherein said step of assessing said level of said each biomarker further comprising measuring the enzymatic activity of at least one enzyme in the blood stream of said animal.

Clause 5. The method of clause 1, wherein said step of computing said index further comprising:

obtaining a discrete value of said level of said each biomarker using at least one discretization criterion for obtaining said discrete value; and

obtaining a product value for each of said biomarker by multiplying said discrete value with a weighing coefficient; and

summing said product value for said each biomarker.

Clause 6. The method of clause 5 further comprising :

determining a classification criterion for obtaining said subgroup; and

adjusting said classification criterion in order to segregate said subgroup.

Clause 7. The method of clause 6 further comprising applying a Receiver Operating Curve analysis to said ranges using said classification criterion.

Clause 8. The method of clause 1, wherein said step of computing said index further comprising:

obtaining a product value by multiplying said discrete value with a weighing coefficient; and

summing said product value for said each biomarker.

Clause 9. The method of clause 8, wherein said step of obtaining said set of ranges of said index value further comprising:

determining a classification criterion for obtaining said subgroup; and

adjusting said classification criterion in order to segregate said subgroup.

Clause 10. The method of clause 8 further comprising applying a Receiver Operating Curve analysis to ranges using said classification criterion.

Clause 11. A system for developing a diagnosis system of ailments in an animal species using a plurality of biomarkers, comprising:

means for assessing a presence of a health affection of a set of health affections in each individual of a group of subjects;

means for assessing a level of each biomarker in a set of biomarkers; and

a computer system for executing computer program code, said program code comprising computer program instructions configured to cause said computer system to:

receive data from said means for assessing said presence of said health affection;

compute an index value for said each individual using said level of said each biomarker;

obtain a set of ranges of said index value, wherein at least one range of said set of ranges comprises at least eighty percent (80%) of a subgroup of individuals, from said group of subjects, that are affected by said health affection from said set of health affections; and output the result of said ranges.

Clause 12. The system of clause 11, wherein said means for assessing said presence of said health affection further comprising means for performing at least one biological test of each of said individual to determine said presence.

Clause 13. The system of clause 11, wherein said means for assessing said level of said each biomarker further comprising means for measuring the concentration of at least one protein in the blood stream of said individual.

Clause 14. The system of clause 11, wherein said means for assessing said level of said each biomarker further comprising means for measuring the enzymatic activity of at least one enzyme in the blood stream of said animal.

Clause 15. The system of clause 11, wherein said computer code configured to cause said computer system to compute said index value further comprises computer program instructions configured to cause said computer system to obtain a discrete value of said level of said each biomarker using at least one discretization criterion to obtain said discrete value.

Clause 16. The system of clause 15 further comprising computer program instructions configured to cause said computer system to:

obtain a product value by multiplying said discrete value with a weighing coefficient; and

sum said product value for said each biomarker.

Clause 17. The system of clause 16, wherein said computer program instructions configured to cause said computer system to obtain said set of ranges of said index value further comprising computer program instructions configured to cause said computer system to determine a Classification criterion for obtaining said subgroup.

Clause 18. The system of clause 17 further comprising computer program instructions configured to cause said computer system to apply Receiver Operating Curve analysis to said ranges using said classification criterion.

Clause 19. The system of clause 11, wherein said computer program instructions configured to cause said computer system to obtain said set of ranges of said index value further comprising computer program instructions configured to cause said computer system to determine a classification criterion for obtaining said subgroup.

Clause 20. The system of clause 19 further comprising computer program instructions configured to cause said computer system to adjust said classification criterion in order to segregate said subgroup.

Clause 21. A method of diagnosing an underlying affection in an animal subject comprising:

obtaining a set of biomarkers and a mapping between a set of ranges of an index value and a plurality of health affections;

assessing a level of each biomarker of said set of biomarkers;

computing said index value using said level of said each biomarker; and

obtaining an underlying health affection by matching said index value to one of the ranges using said mapping.

Clause 22. The method of clause 21, wherein said step of assessing said level of said each biomarker further comprising measuring the concentration of at least one protein in the blood stream of said individual.

Clause 23. The method of clause 21, wherein said step of assessing said level of said each biomarker further comprising measuring the enzymatic activity of at least one enzyme in the blood stream of said animal.

Clause 24. The method of clause 21, wherein said step of computing said index value further comprising:

obtaining a discrete value of said level of said each biomarker using at least one discretization criterion for obtaining said discrete value; and

obtaining a product value by multiplying said discrete value with a weighing coefficient and summing said product value for said each biomarker.

Clause 25. The method of clause 21, wherein said step of computing said index further comprising obtaining a product value by multiplying said discrete value with a weighing coefficient and summing said product value for said each biomarker.

Clause 26. A system of diagnosing an underlying affection in an animal subject comprising:

means for assessing a level of plurality of biomarkers in an individual subject; and

computer system for executing program code, said program code comprising instructions configured to cause said computer system to :

obtain data of a plurality of biomarkers and a mapping data between a set of ranges of an index value and a plurality of health affections;

compute said index value using said level of said plurality of said biomarkers; and

determine an underlying health affection by matching said index value to a range in said set of the ranges using said mapping data.

Clause 27. The system of clause 26, wherein said means for assessing said level of said each biomarker further comprising means for measuring the concentration of at least one protein in the blood stream of said individual subject.

Clause 28. The system of clause 26, wherein said means for assessing said level of said each biomarker further comprising means for measuring the enzymatic activity of at least one enzyme in the blood stream of said individual subject.

Clause 29. The system of clause 26, wherein said computer program further comprises computer program instructions configured to cause said computer system to:

obtain a discrete value of each of plurality of biomarkers using at least one discretization criterion; and

obtain a product value by multiplying said discrete value with a weighing coefficient and summing said product value for said each biomarker.

Clause 30. The system of clause 26, wherein said computer program further comprises computer program instructions configured to cause said computer system to obtain a product value by multiplying said discrete value with a weighing coefficient and summing said product value for said each biomarker.

## Claims

**1.** A method of developing a diagnosis test for the presence of a health affection in an animal species using at least two biomarkers test in tissue samples, comprising the steps of:

defining a set of at least two biomarkers;
assessing a presence of a health affection in a group of subjects in each individual in said group of subjects, forming a subgroup of affected subjects and a subgroup of healthy subjects, and obtaining a tissue/fluid sample from each individual in said group of subjects;
assessing the level of each biomarker of said at least two biomarkers;
computing an index value using said level of said each biomarker, and obtaining a set of ranges of said index value, wherein an affection index range comprises at least eighty percent (80%) of said subgroup of affected subjects; and
providing said affection index range to diagnose a test animal for said health affection by comparing said test animal's biomarker-based index value to said affection index range, and diagnosing said test animal with said health affection if said biomarker-based index value falls within said affection range.

**2.** The method of claim 1, wherein said step of assessing said level of said each biomarker further comprising measuring the concentration/enzymatic activity in a blood sample collected from the blood of said individual.

**3.** The method of claim 1, wherein said step of computing said index further comprising:

obtaining a discrete value of said level of said each biomarker using at least one discretization criterion for defining ranges of said biomarker ranges; and
obtaining a product value for each of said biomarker by multiplying said discrete value with a weighing coefficient; and
summing said product value for said each biomarker.

**4.** The method of claim 3 further comprising determining said index ranges by applying a Receiver Operating Curve analysis to said ranges using said at least eighty percent (80%) criterion.

**5.** The method of claim 3, wherein said step of computing said index further comprising:

obtaining a product value by multiplying said discrete value with a weighing coefficient; and
summing said product value for said each biomarker.

**6.** The method of claim 5 further comprising determining said index ranges by applying a Receiver Operating Curve analysis to ranges using said at least eighty percent (80%) criterion.

7. A system for performing the method as defined in claim 1, said system comprising:

a set of at least two biomarkers;
a group of subjects in each individual is diagnosed with a health affection status and classified within a subgroup of affected subjects, if the individual has the health affection or otherwise in a subgroup of healthy subjects;
testing means for assessing the level of each biomarker of said at least two biomarkers in each individual; and
a computer system for executing computer program code, said program code comprising computer program instructions configured to cause said computer system to:

receive data of said health affection status and biomarker level data from said testing means;
compute an index value using said level of said each biomarker, and obtaining a set of ranges of said index value, wherein an affection index range comprises at least eighty percent (80%) of said subgroup of affected subjects; and
providing an output interface that allows a user to diagnose a test animal for said health affection by comparing said test animal's biomarker-based index value to said affection index range, and diagnose said test animal with said health affection if said biomarker-based index value falls within said affection range.

8. The system of claim 7, wherein said testing means for assessing further comprises measuring the concentration/enzymatic activity of at least one protein/enzyme in a blood sample collected from the blood stream of said said individual.

9. The system of claim 7, wherein said computer code configured to cause said computer system to compute said index value further comprises computer program instructions configured to cause said computer system to obtain a discrete value of said level of said each biomarker using at least one discretization criterion to obtain said discrete value.

10. The system of claim 9 further comprising computer program instructions configured to cause said computer system to:

obtain a product value by multiplying said discrete value with a weighing coefficient; and
sum said product value for said each biomarker.

11. The system of claim 10, wherein said computer program instructions configured to cause said computer system to obtain said set of ranges of said index value further comprising computer program instructions configured to cause said computer system to determine a classification criterion for obtaining said subgroup.

12. The system of claim 11 further comprising computer program instructions configured to cause said computer system to apply Receiver Operating Curve analysis to said ranges using said classification criterion.

13. The system of claim 7, wherein said computer program instructions configured to cause said computer system to obtain said set of ranges of said index value further comprising computer program instructions configured to cause said computer system to determine a classification criterion for obtaining said subgroup.

14. The system of claim 13 further comprising computer program instructions configured to cause said computer system to adjust said classification criterion in order to segregate said affected subgroup.

110
DEFINE TARGET DIAGNOSIS
120
SELECT A SET OF BIOMARKERS
Fig. 1
130
OBTAIN BIOMARKER DATA AND HEALTH STATUS DATA
140
DEFINE BIOMARKER RANGES AND DISCRETIZE BIOMARKER DATA
150
COMPUTE INDEX VALUE FOR EACH SUBJECT
160
APPLY ONE OR MORE SEGREGATION METHODS TO CLUSTER SUBJECTS
NO
170
ARE SAMPLE SUBJECTS MATCHED WITH HEALTH STATUS CATEGORIES ?
YES
180
DEFINE DIAGNOSIS INDEX RANGES

Fig. 2A

210
OBTAIN BIOMARKER DATA FROM A PATIENT
220
DISCRETIZE BIOMARKER DATA AND COMPUTE INDEX VALUE
230
OBTAIN PATIENT'S HEALTH STATUS FROM PRE-ESTABLISHED INDEX RANGES

Fig. 2B

260
264
266
Index Value
STATUS
Affection 1
Affection 2
Affection 3
270

Fig. 3

Number of
Dogs
Age Cohort
60
50
40
30
20
10
0
0-1
1-2
2-3
3-4
4-5
5-6
6-7
7-8
8-9
9-10
10-11
11-12
12-13
13-14
14-15
15-16
16-17
N/A
Age Class
Total
Disqualified
Included

Fig. 4

410
1000
100
10
1
0.1
c-CRP
0.1
1
10
100
1000
TK
Status
Normal
Cancer

# Fig. 5

TK - CONFIRM
Sensitivity
100
80
60
40
20
0
0
20
40
60
80
100
100-Specificity
510

NEOPLASIA INDEX - CONFIRM
Sensitivity
100
80
60
40
20
0
0
20
40
60
80
100
100-Specificity
550

605
Fig. 6
610
DATA ACQUISITION SYSTEMS
630
600
632
620
COMPUTER SYSTEM
634

Europäisches Patentamt
European Patent Office
Office européen des brevets

# EUROPEAN SEARCH REPORT

Application Number
EP 13 19 2071

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br><br>Y | WO 2008/131039 A2 (UNIV TEXAS [US]; MCDEVITT JOHN T [US]; CHRISTODOULIDES NICOLAOS [US];)<br>30 October 2008 (2008-10-30)<br>* the whole document *<br>* in particular: *<br>* claims 1-17 *<br>* paragraph [00118] *<br>* paragraphs [0042], [0060], [0092] - paragraph [0093] *<br>* paragraphs [00111] - [0113], [00153] - paragraph [00155] * | 1,2,7-14<br><br><br><br>3-6 | INV.<br>G01N33/68<br>G06F19/24 |
| X | WO 2008/144316 A1 (UNIV INDIANA RES & TECH CORP [US]; SCRIPPS RESEARCH INST [US]; NICULES) 27 November 2008 (2008-11-27)<br>* the whole document *<br>* in particular: *<br>* claims 1,9 *<br>* paragraphs [00014], [00046] - paragraph [00048] *<br>* paragraphs [00074], [00080] *<br>* paragraph [000132] - paragraph [000134] *<br>* example 1 *<br>* paragraph [000111] - paragraph [000113] * | 1-14 | |
| X<br><br><br><br>Y | WO 2012/021714 A2 (UNIV RICE WILLIAM M [US]; MCDEVITT JOHN T [US]; FLORIANO PIERRE N [US])<br>16 February 2012 (2012-02-16)<br>* claim 1 *<br>* example 5 *<br>* paragraph [0026] * | 1,2<br><br><br><br>3-14 | |

-/--

TECHNICAL FIELDS SEARCHED (IPC)

G01N
G06F

The present search report has been drawn up for all claims

| Place of search | Date of completion of the search | Examiner |
|---|---|---|
| The Hague | 29 January 2014 | Tuynman, Antonin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# EUROPEAN SEARCH REPORT

Application Number
EP 13 19 2071

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2004/058055 A2 (BIOSITE INC [US]; ANDERBERG JOSEPH MICHAEL [US]; BUECHLER KENNETH F [U) 15 July 2004 (2004-07-15) | 1,2 | |
| Y | * the whole document * * claims 1,13,19 * * example 1 * * paragraph [0111] * | 3-14 | |
| X | BLANKENBERG STEFAN ET AL: "Diagnostic value of multimarker testing including myeloperoxidase in patients with acute coronary syndrome results from a multicentre biomarker study", CIRCULATION, vol. 114, no. 18, Suppl. S, October 2006 (2006-10), page 422, XP008167022, & 79TH ANNUAL SCIENTIFIC SESSION OF THE AMERICAN-HEART-ASSOCIATION; CHICAGO, IL, USA; NOVEMBER 12 -15, 2006 ISSN: 0009-7322 | 1,2 | |
| Y | * the whole document * | 3-14 | TECHNICAL FIELDS SEARCHED (IPC) |

The present search report has been drawn up for all claims

| Place of search | Date of completion of the search | Examiner |
|---|---|---|
| The Hague | 29 January 2014 | Tuynman, Antonin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

1

EPO FORM 1503 03.82 (P04C01)

## ANNEX TO THE EUROPEAN SEARCH REPORT ON EUROPEAN PATENT APPLICATION NO. EP 13 19 2071

This annex lists the patent family members relating to the patent documents cited in the above-mentioned European search report.
The members are as contained in the European Patent Office EDP file on
The European Patent Office is in no way liable for these particulars which are merely given for the purpose of information.

29-01-2014

| Patent document cited in search report | Publication date | Patent family member(s) | Publication date |
|---|---|---|---|
| WO 2008131039 A2 | 30-10-2008 | CA 2697357 A1 | 30-10-2008 |
| | | EP 2147115 A2 | 27-01-2010 |
| | | US 2008300798 A1 | 04-12-2008 |
| | | WO 2008131039 A2 | 30-10-2008 |
| WO 2008144316 A1 | 27-11-2008 | US 2011098188 A1 | 28-04-2011 |
| | | WO 2008144316 A1 | 27-11-2008 |
| WO 2012021714 A2 | 16-02-2012 | US 2013130933 A1 | 23-05-2013 |
| | | WO 2012021714 A2 | 16-02-2012 |
| WO 2004058055 A2 | 15-07-2004 | AU 2003300407 A1 | 22-07-2004 |
| | | CA 2511482 A1 | 15-07-2004 |
| | | EP 1588159 A2 | 26-10-2005 |
| | | WO 2004058055 A2 | 15-07-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 8097432 B2